# EUROPÄISCHE PATENTANMELDUNG

(11) **EP 1 112 740 A1**
(43) Veröffentlichungstag der Anmeldung: **04.07.2001**
(21) Anmeldenummer: 99811218.9
(22) Anmeldetag: 30.12.1999
(51) Int. Cl.: A61K 9/48, A61J 3/07, C08K 5/06

(54) **Verwendung eines Polyglycerine enthaltenden wasserlöslichen Mittels**

(71) Anmelder: Greither, Peter, 9533 Kirchberg (CH)
(72) Erfinder: Brocker, Erich R. Dr., 9533 Kirchberg (CH); Maier, Hans-Jürgen, 8636 Wald-Oberholz (CH)
(74) Vertreter: Wenger, René

(57) **Zusammenfassung**

Schmiermittel zum Schmieren von Strängen, vorzugsweise Bändern 4, 4a, aus einem biopolymeren Stoff, insbesondere aus Gelatine, in einem Arbeitsprozess und ein Verfahren zur Herstellung von Weichgelatinekapseln, wobei das Schmiermittel wasserlöslich ist und ein oder mehrere Polyglycerine aus 2 - 28 Monomereinheiten, vorzugsweise 2 - 10 Monomereinheiten, enthält. Vorzug des hydrophilen Schmiermittels ist das Entfallen der Notwendigkeit, an der Kapselhülle verbliebenes Schmiermittel vor der Trocknung der Kapseln 8 zu entfernen sowie das mögliche Recycling der Netzabfälle 14.

## Beschreibung

Gegenstand der vorliegenden Erfindung ist die Verwendung eines mindestens ein Polyglycerin enthaltenden wasserlöslichen Mittels zum Schmieren von Strängen, insbesondere Bändern aus einem biopolymeren Stoff, insbesondere aus Gelatine, in einem wenigstens die Förderung der Stränge beinhaltenden Arbeitsprozess, ein Verfahren zur Herstellung einzeldosierter Darreichungsformen und die Wiederverwendung des mit einem solchen Schmiermittels vermischten Abfalls eines solchen Arbeitsprozesses gemäss den unabhängigen Patentansprüchen 1, 7, 12 und 13.

Kapseln aus Weichgelatine sind ideal als Verpackung für Öle, für lipophile Suspensionen sowie für auf hydrophilen Polymeren basierenden Suspensionen. Insbesondere ermöglichen sie einzeldosierte Darreichungsformen. Die Anwendungen sind vielfältig und umfassen die Gebiete Technik, Nahrung, Gesundheit und Kosmetik. Die effizienteste Herstellung von Weichgelatinekapseln erfolgt nach dem Rotary-Die-Verfahren, das in US 2 288 327 erstmals beschrieben wurde. Die Ausformung der Hülle, deren Befüllung sowie das Verschliessen der Kapsel erfolgen in einem Arbeitsgang an der Kapselfüllstation durch Verschweissen zweier Endlosbänder aus Weichgelatine zwischen zwei als Stanzwerkzeugen rotierenden Formwalzen (vergleiche Bauer, K., ,Die Herstellung von Hart- und Weichgelatinekapseln' in "Die Kapsel", Hrsg. Fahrig, W. et al., Wissenschaftliche Verlagsgesellschaft mbH Stuttgart 1983).

Das Rotary-Die-Verfahren ist aufgrund der damit produzierbaren hohen Stückzahlen das führende Verfahren und ist in seinen Grundzügen im wesentlichen unverändert geblieben; das gilt auch für daraus abgeleitete, ähnliche Verfahren (Norton, Accogel). Einer dieser Grundzüge ist, dass als Schmiermittel für die spannungsfreie Zuführung der Gelatinebänder zur Kapselfüllstation Mineralöle oder Fette (Triglyceride) verwendet werden.

Das Rotary-Die-Verfahren erlaubt in einem ununterbrochenen, einzigen Produktionsschritt die Herstellung von bis zu 100'000 Kapseln pro Stunde. Die als Sol bei ca. 60°C flüssig gehaltene Gelatinemasse mit den darin enthaltenden Hilfsstoffen wird über spezielle Giessvorrichtungen auf zwei Kühltrommeln zu endlosen, gummiartig elastischen Bändern von ca. 0,8 mm Dicke geformt und dann über (geschmierte) Umlenk- und Zwischenrollen zu zwei rotierenden Formwalzen geleitet. Auf den Kühltrommeln vollzieht sich durch Unterschreiten der Gelierungstemperatur von ca. 40°C der Phasenübergang vom Sol zum Gel. Die beiden Gelatinebänder werden in dem schmalen Spalt zwischen den zwei gegenläufig rotierenden Formwalzen zusammengefügt; hier vollzieht sich der Einfüll- und Verschliessvorgang. Die Formwalzen dienen dem Verschweissen der Gelatinebänder und dem Ausstanzen der fertigen, gefüllten Kapsel. Oberhalb bzw. fast zwischen den Formwalzen arbeitet der Füllkeil, über den das Füllgut in die Kapseln gelangt und dessen eingebaute Thermoelemente die Siegelwärme für das Verschweissen der beiden Gelatinebänder entlang des Kapselumrisses generieren.

Das zur Schmierung der Bandzuführung beidseitig auf die Gelatinebänder aufgebrachte Mineralöl oder Triglycerid verhindert ein Kleben an Maschinenteilen, reduziert die Reibung und dient gleichzeitig als Dichtmittel für eine satte Auflage des Füllkeils im Spalt zwischen den beiden Gelatinebändern. Ein luftdichter Abschluss ist entscheidend für das Einpumpen des Füllgutes unter leichtem Druck, wobei die Kapsel ihre eigentliche Form annimmt. Gleichzeitig überträgt das Schmiermittel die Siegelwärme vom Füllkeil auf die Oberfläche der Gelatinebänder und damit auf die zu verschweissenden Oberflächen; der Füllkeil erzeugt eine Temperatur von über 40°C, sodass das Verschweissen der Kapselhälften durch einen kurzzeitigen Übergang vom Gel zum flüssigen Sol bewirkt wird.

Ein Schmiermittel muss also über einen grösseren Temperaturbereich gute Gleiteigenschaften bzw. geeignete Viskositäten aufweisen. Zudem darf das Schmiermittel nicht in die Gelatinematrix diffundieren und dort oder an der Kapseloberfläche unerwünschte Veränderungen hervorrufen.

Als geeignete Schmiermittel wurden bisher Mineralöle oder mittelkettige Triglyceride verwendet. Aus der hydrophoben Natur dieser Schmiermittel leiten sich aber auch Nachteile ab, die bisher bei der Herstellung von Kapseln zwangsläufig in Kauf genommen wurden: Auf der Kapselinnenseite anhaftendes Öl beeinträchtigt die feste Verschweissung der aus einem wasserhaltigen Gelfilm bestehenden Kapselhälften und führt gelegentlich zu Füllgutleckagen. Die Schmiermittel beeinträchtigen die Weiterverarbeitung, insbesondere die Trocknung der frischen Kapseln, die zur Senkung des Wassergehaltes der Gelatinehülle durchgeführt wird. Der dünne Schmiermittelfilm, insbesondere Ölfilm, auf der Aussenseite führt zum Verkleben der Kapseln während der Trocknung.

Der Schmiermittelfilm muss daher in einem zusätzlichen Arbeitsschritt entfernt werden. Verwendet werden vor allem Waschbäder mit organischen Lösungsmitteln wie z.B. Methylenchlorid, 1,1,1-Trichlorethan, 1,1,2-Trichlorethylen, Naphta, Isopar, Freone, etc. Der Einsatz von z.T. toxischen, umweltschädigenden, leichtflüchtigen organischen Lösungsmitteln ist jedoch unerwünscht. Die Entfernung des Schmiermittelfilms mit diesen Lösungsmitteln führt zu hohen Kosten für die Rückstandsanalytik im Endprodukt und regulatorischen Schwierigkeiten (Einsatzverbot, Umweltauflagen und nicht eliminierbare Kontamination des Lösungsmittels durch die aktiven Stoffe der Darreichungsform bei der Wiederaufbereitung). Vor allem ist aber der zusätzliche Arbeitsschritt des Waschens unerwünscht, da der damit verbundene Aufwand proportional mit der produzierten Menge ansteigt.

Ein weiterer Nachteil der Verwendung dieser im Stand der Technik bekannten Schmiermittel liegt in der Kontamination der sogenannten Netzabfälle, d.h. der nach Ausstanzen der Kapseln überbleibenden perforierten Gelatinebänder, durch das Schmiermittel. Die Netzabfälle betragen etwa 30 - 60% der zum Giessen der Gelatinebänder ursprünglich eingesetzten Gelatinemasse. Die Netzabfälle können bisher nicht im selben oder ähnlichen Verfahren wiederverwertet werden. Die Zusammensetzung der Gelatinemasse wird durch enthaltende Spuren von Öl, das nicht vollständig abgetrennt werden kann, nachteilig verändert. Beim Einschmelzen zum Sol ergibt sich ein öliges zwei-Phasen-Gemisch, das ein kontinuierliches, störungsfreies Giessen der Gelbänder verunmöglicht.

Die Menge der Netzabfälle, d.h. des als Ausschuss verworfenen Materials, erhöht wesentlich die Produktionskosten. Zusätzlich kostensteigend ist der besondere Aufwand mit dem der Netzabfall entsorgt wird. Die Wiederverwendung der Netzabfälle im Produktionsprozess oder ähnlicher Produkte, bei denen ebenfalls Spuren von Mineralöle oder Triglyceriden nicht tolerierbar sind, ist daher aus Kostenerwägungen sehr wünschenswert.

Alternativverfahren zur Herstellung von Weichgelatinekapseln, wie das "Drop and Blow-Verfahren", erreichen nicht die Produktionsleistung des Rotary-Die-Prozesses oder sind nicht gleich robust in der Prozessführung, was sich z.B. im Einstellen der Temperatur und Viskosität der Gelatinemasse, oder der mangelnden Verarbeitung von hochviskosen Füllgütern oder Pasten negativ bemerkbar macht.

Aufgabe der vorliegenden Erfindung ist es, die Nachteile des Bekannten zu vermeiden. Insbesondere besteht die Aufgabe darin ein Schmiermittel bereitzustellen, das die Wiederverwendung damit kontaminierter Netzabfälle erlaubt. Weiterhin besteht die Aufgabe darin, einen abschliessenden Waschschritt überflüssig zu machen oder gegebenenfalls ohne Einsatz von organischen Lösungsmitteln das Schmiermittel von der Kapseloberfläche zu entfernen. Es ist eine weitere Aufgabe der vorliegenden Erfindung die Verschweissung der auf einer wasserhaltigen Gelatinematrix beruhenden Kapselhälften nicht zu beeinträchtigen. Weiterhin soll ein Verfahren zur Verfügung gestellt werden, mit dem die Netzabfälle wiederverwendet und zu weiteren Produkten geformt werden können.

Diese Aufgaben werden gelöst durch die kennzeichnenden Teile der unabhängigen Ansprüche.

Insbesondere werden sie gelöst durch die Verwendung eines mindestens ein Polyglycerin enthaltenden wasserlöslichen Mittels zum Schmieren von Strängen aus mindestens einem biopolymeren Stoff in einem wenigstens die Forderung der Stränge beinhaltenden Arbeitsprozess. Insbesondere soll der Arbeitsprozess Bestandteil des Prozesses zur Herstellung von Weichkapseln sein.

Es wurde überraschend gefunden, dass hydrophile Polyglycerine als Schmiermittel bei der Herstellung von Formkörpern, insbesondere Weichgelatinekapseln nach dem Rotary-Die-Verfahren, oder einem anderen, ähnlichen Verfahren zur Herstellung von Formkörpern aus einem Biopolymer geeignet sind.

Das Mittel zum Schmieren ist wasserlöslich bzw. wasserdispergierbar und umfasst ein oder mehrere Polyglycerine, vorzugsweise aus 2 bis 28 Monomereinheiten. Während viele hydrophile Stoffe wie beispielsweise Glycerin, Polyethylenglycole oder Propylenglycole als Schmiermittel ungeeignet sind, da sie entweder ungenügend schmieren, ungenügend dichten oder eine unerwünschte Veränderung der Gelmatrix bewirken, verbinden Polyglycerine als Schmiermittel sehr gute Gleiteigenschaften mit einem neutralem Verhalten gegenüber der wasserhaltigen Matrix, insbesondere gegenüber der Gelatinematrix. Es ist möglich, nebst Gelatine auch andere zu Bändern oder Strängen verarbeitete Biopolymere, z.B. Stärke, Stärkederivate, Cellulose, Cellulosederivate, Galactomannane, Gummiarten, Agar-Agar, und andere sowie Mischungen dieser Biopolymere zu verwenden.

Das äusserlich auf die Kapseln oder andere Formkörper aufgebrachte Polyglycerin kann durch Wasser oder physiologisch unbedenkliche Stoffe wie Ethanol leicht entfernt werden. Bei Verwendung des erfindungsgemässen Schmiermittels ist der Waschschritt, d.h. die Entfernung des Schmiermittels von der Formkörperhülle, nur noch fakultativ. Das Abwaschen des Films vor oder während der Trocknung ist nicht mehr unbedingt erforderlich. Die Polyglycerine als hydrophile Substanzen können bei geeigneter Kettenlänge von der Oberfläche der Gelmatrix absorbiert werden bzw. diffundieren in die Gelmatrix wenigstens oberflächlich. Eine Molekülgrösse des Polyglycerins von 2 bis 10 Monomereinheiten ist besonders geeignet. Mit einem dünnen Film von Polyglycerin bedeckte Kapseln verkleben während oder Trocknung nicht, vermutlich in Folge der besseren Benetzung der Kapseloberfläche bzw. der Oberflächenspannung, was unnötige Produktionsverluste vermeidet. Insbesondere dem auf die Kapselbefüllung nachfolgenden Trocknungsvorgang kommt zugute, dass das leicht hygroskopische Polyol Wasser aus der Kapselhülle zieht und generell dem Trocknungsprozess keine hydrophobe Diffusionsbarriere entgegensetzt.

Die Polyglycerine zeigen überragende Eigenschaften als Gleitmittel, dichten im Bereich des warmen Füllkeils gut ab und fördern die thermomechanische Verschweissung der Kapselhälften. Insbesondere bei hydrophilen Füllgütern, bei denen die Wärmeübertragung durch ein konventionelles, hydrophobes Schmiermittel zu ungenügender Schweissnahtbildung und somit zu Leckagen führen kann, ist ein hydrophiles Schmiermittel auf Polyglycerinbasis überlegen.

Ein weiterer Vorteil bei der Verwendung von Polyglycerin in Schmiermitteln besteht darin, dass die oberflächlich mit Schmiermittel überzogenen Netzabfälle, bei der Herstellung von Formkörpern, insbesondere Weichgelatinekapseln einer Wiederverwendung zugeführt werden können. Ein hydrophiles Schmiermittel bildet beim Einschmelzen mit dem wasserhaltigen Polymer-Sol eine homogene Phase, die für eine Wiederverwertung z.B. durchaus im gleichen Prozess, zur Verfügung steht. Anhaftende Reste von Füllgut sind tolerierbar, wenn der Netzabfall zu Formkörpern recycliert wird, der mit dem gleichen Füllgut befüllt wird.

Polyglycerine sind als Weichmacher beispielsweise in Gelatinefilmen bekannt; insofern die thermischen oder mechanischen Eigenschaften der recyclierten Netzabfälle, beispielsweise von Gelatine, durch die Polyglycerine überhaupt eine Veränderung erfahren, so erfolgt diese Veränderung in vorhersagbarer und erwünschter Weise. Das durch die Recyclierung der Netzabfälle eingebrachte Polyglycerin bewegt sich mengenmässig maximal bei einigen Prozent bzw. kann so eingestellt werden. Durch Beimischen eines Teils frischer Biopolymermasse mit geringerem Weichmacheranteil, der sich auch aus anderen Weichmachern als Polyglycerin zusammensetzen kann, wird dieser Effekt zudem neutralisiert. Auch können Rakels eingesetzt werden, um die an den Netzabfällen anhafteten Reste von Polyglycerin durch Abstreifen unter den Bereich von ca. 1% zu senken.

Ein Strang oder Band auch im Sinne der vorliegenden Erfindung, wird durch Giessen, Blasen, Extrudieren, Tropf- bzw. Tauchverfahren hergestellt. Die Biopolymermasse kann dabei z.B. durch Gel-Sol-Übergang die notwendige Viskosität bzw. Festigkeit annehmen oder bereits vorher besessen haben (z.B. bei extrudierbaren Mischungen).

Polyglycerine im Sinne der vorliegenden Erfindung sind Oligomere mit höchstens 28 Monomereinheiten Glycerin HO-CH₂-CH(OH)-CH₂-OH, die z.B. in einer Kondensationsreaktion aus Glycerol unter Ausbildung eines Polyethers erhalten werden. Herstellungstechnisch fallen die Oligomeren dabei in Gemischen unterschiedlicher Kettenlängen an. Deshalb bedeutet die Angabe eines bestimmten Oligomeres, z.B. Decaglycerin eine Mischung, welche mehrheitlich dieses bestimmte Oligomer enthält. So können auch noch Anteile an Di-, Tri- und weiteren Glycerinen vorhanden sein.

Es sind aber auch andere Synthesewege möglich, z.B. die Umsetzung eines Startmoleküls Glycerin mit dem entsprechenden (Hydroxy-Methyl)-Oxiran. Die Kondensation von Glycerol führt mit zunehmender Zahl verknüpfter Monomere zu einer wachsenden Anzahl beliebig verzweigter Konstitutionsisomere aufgrund der dreifachen Funktionalität des Glycerolmonomers. Es ist auch möglich, die Kondensationspolymerisation mit einem partiell durch Schutzgruppen derivatisierten Glycerin durchzuführen, wobei die Monomeren nur bifunktionell sind und sich unverzweigte Polyether ergeben.

Polyglycerine im Sinne der vorliegenden Erfindung sind durch ihre Molmasse charakterisiert, die der Zahl der darin miteinander über Etherbindung verknüpften Glycerolmonomeren entspricht. (Lexikon der Hilfsstoffe, P. Fiedler, Editio Cantor Verlag Aulendorf, 1996). Während das Monomere Glycerin rein oder als Mischung mit anderen organischen Stoffen nur mässig gut als Gleitmittel geeignet ist, sind dessen höhere Homologen vom Diglycerin an als Schmiermittel im Rotary-Die-Verfahren oder einem ähnlichen Arbeitsprozess (s.o.) gut geeignet. Polyglycerine im Sinne der vorliegenden Erfindung entstehen durch Verknüpfung von 2 bis 28 Glycerolmonomeren. Vom Glycerin unterscheiden sie sich durch eine mit der Kettenlänge zunehmende Viskosität und Solvatationsvermögen. Ein Polyglycerine im Sinne der vorliegenden Erfindung ist weiterhin bei Raumtemperatur eine Flüssigkeit und ist bei Raumtemperatur mit Wasser in jedem Verhältnis mischbar. Polyglycerine mit mehrheitlich Oligomeren eines Molekulargewichts von 750 g/mol ist noch flüssig. Eine bevorzugte Ausführungsform im Sinne der vorliegenden Erfindung ist Decaglycerin.

Solange die Eigenschaft als Schmiermittel bzw. die Wasserlöslichkeit im Sinne des vorher Gesagten nicht beeinträchtigt wird, können die Polyglycerine auch partiell derivatisiert, z.B. verestert sein. Es können auch Gemische von Polyglycerinen und Polyglycerinderivaten, insbesondere Polyglycerinester als Schmiermittel eingesetzt werden.

Ein weiterer Vorzug des erfindungsgemässen Schmiermittels ergibt sich dann, wenn eine Kapselnachbehandlung erfolgt, bei der durch Filme oder Überzüge spezielle Eigenschaften wie Zeit- oder pH-kontrollierte Löslichkeit im Magen erreicht werden sollen. Für diese Anwendungen werden wasserbasierte Überzüge bevorzugt, um ein Handling mit leichtflüchtigen organischen Lösungsmitteln zu vermeiden. Reste von Polyglycerin z. B. auf der Kapseloberfläche sind mit derartigen Überzügen kompatibel. Es ist auch möglich, derartige Überzüge bereits dem Schmiermittel beizumischen, z.B. wässrige Mischungen von Polyglycerin und Hydroxymethylcellulose oder anderer Stärke- und Cellulosederivate, soweit sich dadurch nicht die Schmiermitteleigenschaften wesentlich verändern. In einer weiteren bevorzugten Ausführungsform umfasst das Schmiermittel deshalb ein Cellulosederivat, vorzugsweise ein als magensaftresistenter Überzug geeignetes Cellulosederivat. Ein Beispiel für ein magensaftresistenten Überzug aus einem Cellulosederivat ist z.B. Hydroxy-propylmethyl-cellulose-acetatsuccinat (HPMCAS). HPMCAS ist in wässriger Lösung nur bei einem pH > 5 löslich.

In einer bevorzugten Ausführungsform enthält das Schmiermittel zusätzlich strukturverändernde Substanzen für das biopolymere Material, z.B. Formaldehyd oder Xylose für Gelatine.

Bei Zugabe von Hydroxymethylpropylcellulosephtalat, Cellulosephtalat, Emulsionen aus Methylmethacrylat ist es auch möglich für den nachfolgenden Lackauftrag einen Primer zu schaffen.

Ein Schmiermittel im Sinne der vorliegenden Erfindung umfasst zusätzlich Wasser, mit Wasser oder Polyglycerin homogen mischbare organische Stoffe, in Polyglycerin oder Wasser, optional unter Verwendung eines Emulgators, gelöste organische Stoffe, vorzugsweise Cellulosederivate, oder mit Wasser homogen mischbare organische Lösungsmittel, allein oder in beliebiger Kombination.

In einer weiteren bevorzugten Ausführungsform umfasst das Schmiermittel aliphatische oder cyklische Alkohole, Glycol, Glycerin, Propylenglycol oder Polypropylenglycol, Ethylenglycol oder ein Polyethylenglycol alleine oder in Kombination. Die Beimischung dieser hydrophilen Substanzen erlaubt es, die Eigenschaft eines auf Polyglycerin basierenden Schmiermittels optimal auf die Eigenschaften des verwendeten Biopolymers oder auf die spezifischen Produktionsanforderungen abzustimmen. Die Vorteile der hydrophilen Polyglycerine als Schmiermittel werden dabei gewahrt. Insbesondere Komponenten wie Polyalkenylglycole diffundieren bei hinreichender Kettenlänge nicht in die Gelmatrix.

Biopolymere wie Cellulosederivate können als zusätzliche, rheologieeinstellende Additive dienen. In der Galenik sind vielfach wasserlösliche, durch Veretherung mit (Hydroxy-Alkoxygruppen oder durch Veresterung z.B. mit Phthalsäure derivatisierte Cellulosen gebräuchlich.

Vorzugsweise beträgt der Mischungsanteil des Polyglycerins am Schmiermittel mindestens 5%, in einer bevorzugten Ausführungsform mindestens 15 %, und in einer am meisten bevorzugten Ausführungsform mindestens 40% des Gesamtgewichts.

In einer weiteren bevorzugten Ausführungsform besteht das Polyglycerin oder Polyglyceringemisch des erfindungsgemässen Schmiermittels aus unverzweigten Kettenmolekülen. Derartige Kettenmoleküle zeigen besonders gute Gleitmitteleigenschaften in einem weiten Temperaturbereich zwischen ca. 5 bis 50°C und verbinden diese aufgrund leichter Strukturviskosität mit guten Dichtungseigenschaften im Bereich des Füllkeils. Die Viskosität steigt linear mit der Kettenlänge an; die Zugabe oder alleinige Verwendung von unverzweigten Polyglycerine zu den erfindungsgemässen Schmiermitteln erlauben damit insbesondere in Mischung mit den anderen, oben genannten Stoffen die gezielte Herstellung eines Schmiermittels geeigneter Viskosität.

Ein weiterer Gegenstand der vorliegenden Erfindung ist ein Verfahren zur Herstellung von Dosierkapseln unter Verwendung eines ein Polyglycerin oder Polyglyceringemisch mit 2 - 28 Monomereinheiten, vorzugsweise 2 - 10 Monomereinheiten, enthaltenden wasserlöslichen Schmiermittels gemäss dem oben Gesagten in einem die Förderung eines Stranges, vorzugsweise eines Bandes, eines Biopolymers beinhaltendem Arbeitsprozess, umfassend die Schritte des Giessens eines kontinuierlichen ersten Gelstranges und mindestens eines kontinuierlichen zweiten Gelstranges, des Auftragens eines Schmiermittels auf mindestens eine Seite mindestens eines Gelstranges, des Vereinigens mindestens eines ersten und eines zweiten Gelstranges zu Geltaschen und des Einfüllens eines Füllgutes unter Ausbildung einer Kapselform und schliesslich die Trocknung der Kapseln.

Ein durch diese Schritte beschriebenes Herstellungsverfahren sind z.B. das Rotary-Die-Verfahren und das Accogel-Verfahren. Beide Verfahren erlauben die Herstellung von rundum verschlossenen Formkörpern, insbesondere Kapseln, aus einer Vielzahl von filmbildenden Biopolymermischungen, wie z.B. Gelatine. Es ist aber auch möglich, derartige Kapseln im Reciprocating-Die-Verfahren (Norton) oder einem anderen, kontinuierlichen Stanzverfahren unter Verwendung von Gelbändern oder -strängen herzustellen.

Ein weiterer Vorteil des Verfahrens ist das Entfallen eines zusätzlichen Reinigungsschritts zum Entfernen des den Formkörper anhaftenden Schmiermittels. Das wasserlösliche Schmiermittel behindert die Trocknung nicht und verhindert das Verkleben der Kapseln. Trocknung im Sinne der vorliegenden Erfindung kann z.B. Umlufttrocknung sein, es kann sich aber auch um Trocknung z.B. in Gegenwart eines Trockenmittels bei Unterdruck u.ä. handeln. Die Trocknung dient in jedem Falle der Senkung des Lösungsmittelgehalts der Gelmatrix, vorzugsweise des Wassergehaltes; der Formkörper wird dadurch verfestigt.

Erfindungsgemässe Biopolymermischungen, vorzugsweise Gelatinemischungen, enthalten üblicherweise Weichmacher, z.B. Glycerin, Polyglycerin, Sorbitol, Propylglycol, Ditmethylpolysiloxane und Ethylenglycol. Es ist möglich, filmbildende Mischungen zu verwenden, die aus Gelatine, Cellulose oder Cellulosederivat, Stärke oder einem anderen Biopolymer, Mischungen daraus oder wenigstens zum Teil aus einem Derivat oder Abbauprodukt eines Biopolymers bestehen. Vorzugsweise handelt es sich um eine Gelatine- oder gelatinehaltige Mischung.

Es ist auch möglich, die Kapseln vor oder während der Trocknung von den auf der Kapselhülle verbliebenen Resten des Schmiermittels zu reinigen. Vorteil dabei ist, das keine organischen Lösungsmittel verwendet werden müssen. Das Schmiermittel ist von der Oberfläche der Kapseln durch unschädliche, hydrophile Flüssigkeiten wie z.B. (kaltes) Wasser, Alkohole, ein Gemisch dieser Stoffe, oder aber durch ein festes Bindemittel z.B. Baumwolltücher leicht zu entfernen. Derartige Methoden zur Entfernung des auf der Kapseloberfläche zurückbleibenden dünnen Films des Schmiermittels sind Gegenstand bevorzugter Ausführungsformen des erfindungsgemässen Verfahrens.

Ein weiterer Gegenstand der vorliegenden Erfindung ist die wenigstens teilweise Verwendung der bei der Herstellung von Weichgelatinekapseln in einem kontinuierlichen Verfahren, nach Ausstanzen der Kapseln aus einem oder mehreren mit Schmiermittel bestrichenen Gelatinebändern anfallenden Gelatineabfälle als Ausgangsmaterial zur Herstellung von Gelatinebänder, wobei das Schmiermittel wasserlöslich oder wasserdispergierbar ist und ein oder mehrere Polyglycerine vorzugsweise mit 2 bis 28 Monomereinheiten umfasst.

Ein weiterer Gegenstand der vorliegenden Erfindung ist die Verwendung von biopolymeren Materialien, vorzugsweise Gelatine, enthaltenden ein wasserlösliches oder wasserdispergierbares Schmiermittel, welches ein oder mehrere Polyglycerine umfasst vorzugsweise mit 2 bis 28 Monomereinheiten, und als Abfall eines ersten Arbeitsprozesses zur Herstellung eines Formkörpers anfällt, in einem zweiten Arbeitsprozess.

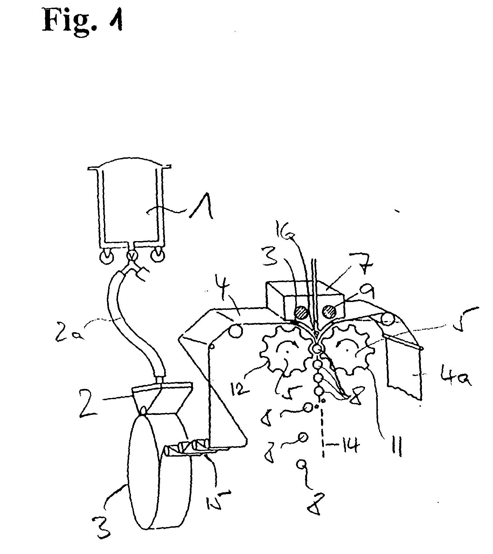

Figur 1 zeigt schematisiert das Rotary-Die-Verfahren.

Das Rotary-Die-Verfahren stützt sich auf eine Verkapselungsmaschine mit rotierenden Formwalzen 5,5a . Charakteristisch ist der einstufige Fertigungsgang. Die Herstellung der Kapselhülle, ihre Befüllung sowie das Ausstanzen der Kapseln erfolgen in einer einzigen Maschine, wie in der Figur 1 dargestellt.

Aus einem Gelatinevorratsbehälter 1 der das Gelatine-Sol mit Zuschlagstoffen wie z.B. Weichmachern bei 60 - 70 °C in flüssiger Form enthält, wird über eine spezielle Bandgiessvorrichtung 2 (mit Zuleitung 2a) auf einer Kühltrommeln 3 ein endloses gummiartig, elastisches Band von im Mittel ungefähr 0,8 mm Dicke gegossen. Auf der Kühltrommel 3, die in einem Temperaturbereich von 5 bis 25°C arbeitet, vollzieht sich der Übergang zum Gel. Die genaue Kühltemperatur ist dabei ein wichtiger Verfahrensparameter, um die Eigenschaften des Gelbandes 4,4a d.h. die Elastizität zu steuern. Die Kühltrommel 3 verfügt über glatte Oberflächen, die überlicherweise nicht geschmiert werden. Das auf der Kühltrommel 3 erzeugte Gelatineband 4,4a wird dann über mehrere Schmierwalzen 15 und Umlenkwalzen spannungsfrei zu den Formwalzen 5,5a geführt. Die Schmierwalzen 15 verfügen z.B. über eine saugfähige Oberfläche und können von einem inneren Hohlraumvolumen mit Schmiermittel gespeist werden. Es sind aber auch andere Mittel für das Aufbringen des Schmiermittels auf das Gelatineband, wie Tauchen Sinterwalzen, etc. möglich.

Das Gelatineband 4 wird mit einer Geschwindigkeit von ungefähr 1 - 2 m/min transportiert, wobei einzelne Walzen mit Antriebsmitteln versehen sein können.

Oberhalb bzw. zwischen den Formwalzen 5,5a arbeitet der Füllkeil 7, über den das Füllgut 16, in die Kapseln 8 gelangt und dessen eingebaute Heizelemente 9 die Siegelwärme für das Verschweissen der aus den zwei Gelatinebändern 4, 4a gebildeten Kapselhälften abgeben. Aus den Gelatinebändern 4a,4 bilden die mit Aussparungen 12 versehenen, gegenläufigen Formwalzen 5, 5a durch Zusammenpressen flache, taschenartige Gebilde. Die Aussparungen 12 entsprechen dem Kapselumriss, entlang dem der Gelbänder 4, 4a verschweisst werden.

Kurz vor dem vollständigen Verschliessen der durch diese Aussparung 12 definierten Geltasche wird das Füllgut 16, durch den Füllkeil 7 in diese Taschen gedrückt, wobei die Kapsel 8 ihre eigentliche Form annimmt. Der Walzendruck auf die Ränder 11 der Aussparung 12 bewirkt ausserdem das Ausstanzen der Kapseln 8. Der Spalt 3 zwischen den zusammenlaufenden Gelatinebändern 4, 4a und dem Füllkeil 7 wird durch aufgestautes Schmiermittel abgedichtet. Diese Dichtungsfunktion ist essentiell für die korrekte Befüllung der Kapsel 8. Das Schmiermittel überträgt im Spalt 3 die Wärme der Heizelemente 9 vom Füllkeil 7 auf die Oberfläche der zu verschweissenden Gelatinebänder 4, 4a. Das Schmiermittel muss also in einem Temperaturbereich von 20°C (auf den Walzen 15) bis zur Heiztemperatur von ca. 40° (am Füllkeil 7) geeignete Schmiereigenschaften aufweisen. Ein hydrophiles Schmiermittel interferiert weniger mit dem Verschweissen der feuchten Matrix der Gelatinebänder4, 4a. Das nach dem Ausstanzen der Kapseln verbleibende Gelatineband, das als Netzabfall 14 bezeichnet wird, ist auf der Oberfläche mit Schmiermittel kontaminiert. Konventionelle Schmiermittel (Mineralöle bzw. Triglyceride), erlauben die Wiederverwendung des Netzabfalls 14 durch Einschmelzen zum Sol nicht. Polyglycerine als hydrophiles Schmiermittel mischen sich hingegen beim Einschmelzen homogen mit der Gelatinemasse und beeinflussen die Eigenschaften des daraus hergestellten Gelatinebandes 4, 4a nicht negativ bzw. in vorhersagbarer Weise und Ausmass, da Polyglycerin aus dem Stand der Technik als Weichmacher für Weichgelatinekapseln bekannt ist. Es ist z.B. möglich, die Gelatinemasse im Vorratsbehälter 1 teilweise aus Netzabfällen 14 und frischem Material mit geringerem Weichmacheranteil anzumischen. Dadurch machen sich die durch das Schmiermittel resultierenden Polyglycerinbeimengungen nicht störend bemerkbar.

### Beispiel 1

Ein erfindungsgemässes Schmiermittel wird hergestellt durch Mischen von drei Massenteilen Dekaglycerin (Hersteller: Sakamoto Yakuhin Kogyo C. Ltd.) mit einem Massenteil Wasser. Unter Verwendung diese Schmiermittels wurden Weichgelatinekapseln in einem Rotary-Die-Verfahren hergestellt. Die erhaltenen Kapseln zeigen eine sehr gut ausgebildete Naht, lassen sich schnell trocknen, zeigen Formstabilität und kleben nicht.

### Beispiel 2

Ein erfindungsgemässes Schmiermittel wird hergestellt durch Mischen von zwei Massenteilen Hexaglycerin mit zwei Massenteilen Wasser. Unter Verwendung dieses Bandgleitmittels wurden Weichgelatinekapseln im Rotary-Die-Verfahren hergestellt. Die erhaltenen Kapseln zeigen wiederum eine sehr gut ausgebildete Naht, lassen sich schnell trocknen und zeigen Formstabilität.

### Beispiel 3

Ein erfindungsgemässes Schmiermittel wird hergestellt von drei Massenteilen Hexaglycerin mit einem Massenteil PEG 400 (400 = Molekulargewicht). Unter Verwendung dieses Bandgleitmittels wurden Weichgelatinekapseln hergestellt nach dem Rotary-Die-Verfahren. Die erhaltenen Kapseln zeigen eine sehr gut ausgebildete Naht, lassen sich schnell trocknen und zeigen Formstabilität.

## Patentansprüche

1. Verwendung von einem mindestens ein Polyglycerin, vorzugsweise aus 2 bis 28 Monomereinheiten, enthaltenden wasserlöslichen Mittels zum Schmieren von Strängen, insbesondere Bändern, aus mindestens einem biopolymeren Stoff, insbesondere aus Gelatine, in einem wenigstens die Förderung der Stränge beinhaltenden Arbeitsprozess, insbesondere in einem Prozess zum Herstellen von Kapseln.

2. Mittel zum Schmieren gemäss Anspruch 1, dadurch gekennzeichnet dass das Mittel zum Schmieren zusätzlich Wasser, mit Wasser oder Polyglycerin homogen mischbare organische Stoffe, in Polyglycerin oder Wasser, optional unter Verwendung eines Emulgators, gelöste organische Stoffe, vorzugsweise Cellulosederivate, oder mit Wasser homogen mischbare organische Lösungsmittel, allein oder in beliebiger Kombination, umfasst.

3. Mittel zum Schmieren gemäss Anspruch 2, dadurch gekennzeichnet, dass die mit Wasser oder Polyglycerin mischbaren organischen Stoff aliphatische und/oder cyclische Alkohole, Glycol, Glycerin, Propylenglycol und/oder Polypropylenglycol, Ethylenglycol oder Polyethylenglycol, allein oder in Kombination, umfasst.

4. Mittel zum Schmieren gemäss Anspruch 2, dadurch gekennzeichnet, dass zusätzliche strukturverändernde Substanzen für den biopolymeren Stoff vorhanden sind wie z.B. Formaldehyd und Xylose.

5. Verwendung eines Mittels zum Schmieren gemäss einem der vorhergehenden Ansprüche für die Herstellung einer vorzugsweise magensaftresistent überzogenen Kapsel, dadurch gekennzeichnet, dass das Schmiermittel ein Cellulosederivat umfasst, vorzugsweise ein als magensaftresistenter Überzug geeignetes Cellulosederivat .

6. Mittel zum Schmieren gemäss einem der vorhergehenden Ansprüche, dadurch gekennzeichnet dass der Mischungsanteil des Polyglycerins am Schmiermittel mindestens 5% des Gesamtgewichts, vorzugsweise wenigstens 15% des Gesamtgewichts, am bevorzugtesten wenigstens 40 % des Gesamtgewichts, beträgt.

7. Mittel zum Schmieren gemäss einem der vorhergehenden Ansprüche, dadurch gekennzeichnet dass das Polyglycerin unverzweigt ist.

8. Mittel zum Schmieren wenigstens eines Stranges, insbesondere eines Bandes, aus einem biopolymeren Stoff, insbesondere aus Gelatine, in einem wenigstens die Förderung des Stranges beinhaltenden Arbeitsprozess, dadurch gekennzeichnet, dass das Schmiermittel wasserlöslich ist und ein oder mehrere Polyglycerine aus 2 bis 28 Monomereinheiten und ein Cellulosederivat umfasst, vorzugsweise ein als magensaftresistenter Überzug geeignetes Cellulosederivat.

9. Mittel gemäss Anspruch 8, dadurch gekennzeichnet dass der Mischungsanteil des Polyglycerins am Schmiermittel mindestens 5% des Gesamtgewichts, vorzugsweise mindestens 15% des Gesamtgewichts und am bevorzugtesten 40% des Gesamtgewichts beträgt.

10. Verfahren zur Herstellung von Dosierkapseln unter Verwendung eines ein Polyglycerin oder Polyglycerin-Gemisch mit 2 bis 28 Monomereneinheiten, vorzugsweise 4 bis 10 Monomereneinheiten, enthaltenden wasserlöslichen Schmiermittels in einem die Förderung eines Stranges, vorzugsweise eines Bandes, aus einem Biopolymer beinhaltenden Arbeitsprozess, umfassend die Schritte
- Giessen eines kontinuierlichen ersten Gelstranges und mindestens eines kontinuierlichen zweiten Gelstranges,
- Auftragen eines Schmiermittels auf mindestens eine Seite mindestens eines Gelstranges
- Vereinigen mindestens eines ersten und eines zweiten Gelstranges zu Geltaschen und Einfüllen eines Füllgutes unter Ausbildung einer Kapselform.
- Trocknung der Kapseln

11. Verfahren gemäss Anspruch 10, dadurch gekennzeichnet, dass in einem zusätzlichen Verfahrensschritt vor der Trocknung das Entfernen des Schmiermittels durch Wasser, einen Alkohol oder ein Gemisch dieser Stoffe vorgenommen wird.

12. Verfahren gemäss Anspruch 10, dadurch gekennzeichnet, dass in einem zusätzlichen Verfahrensschritt vor oder während der Trocknung das Entfernen des Schmiermittels durch ein festes Bindemittel, vorzugsweise Baumwollgewebe, vorgenommen wird.

13. Verwendung der bei der Herstellung von Weichgelatinekapseln in einem kontinuierlichen Verfahren, vorzugsweise im Rotary-Die-Verfahren, nach Ausstanzen der Kapseln aus einem oder mehreren mit Schmiermittel beschichteten Gelatinebändern anfallenden Gelatineabfälle wenigstens teilweise als Ausgangsmaterial zur Herstellung besagter Gelatinebänder, wobei das Schmiermittel wasserlöslich ist und mindestens ein Polyglycerin aus 2 bis 28 Monomereinheiten enthält.

14. Verwendung der ein biopolymeres Material, vorzugsweise Gelatine, enthaltenden und mit einem wasserlöslichem Schmiermittel umfassend mindestens ein Polyglycerin aus 2 bis 28 Monomereinheiten vermischten Abfälle eines ersten Arbeitsprozesses zur Herstellung eines Erzeugnisses, vorzugsweise eines Formkörpers, in einem zweiten Arbeitsprozess.
